# EUROPÄISCHE PATENTSCHRIFT

(11) **EP 3 453 352 B1**
(45) Veröffentlichungstag und Bekanntmachung des Hinweises auf die Patenterteilung: **11.09.2024**
(21) Anmeldenummer: 18185937.2
(22) Anmeldetag: 27.07.2018
(51) Int. Cl.: A61B 18/14, A61B 18/00

(54) **MEDIZINISCHES INSTRUMENT ZUM ABTRAGEN VON GEWEBE MITTELS EINER HF-ELEKTRODE MIT DER FUNKTION EINER KONTROLLIERTEN DISTALEN WINKELAUSRICHTUNG**
MEDICAL INSTRUMENT FOR REMOVING TISSUE BY MEANS OF A HIGH FREQUENCY (HF) ELECTRODE HAVING THE FUNCTION OF A CONTROLLED DISTAL ANGULAR ORIENTATION
INSTRUMENT MÉDICAL PERMETTANT D'ENLEVER DES TISSUS AU MOYEN D'UNE ÉLECTRODE HAUTE FRÉQUENCE POURVUE DE FONCTION D'UNE ORIENTATION ANGULAIRE DISTALE CONTROLLÉE

(30) Priorität: 05.09.2017 DE 102017120341
(43) Veröffentlichungstag der Anmeldung: 13.03.2019
(73) Patentinhaber: Karl Storz SE & Co. KG, 78532 Tuttlingen (DE)
(72) Erfinder: Doll, Frank, 78532 Tuttlingen (DE); Hermle, Rainer, 78532 Tuttlingen (DE); Löffler, Oliver, 78532 Tuttlingen (DE); Rehbein, Stefan, 78532 Tuttlingen (DE); Nagele, Dr. Udo, 6300 Wörgl (AT); Wittke, Uwe, 78532 Tuttlingen (DE)

(56) Entgegenhaltungen:
- EP-A1- 1 974 683
- DE-A1- 10 354 830
- US-A- 5 976 129
- US-B1- 6 231 591

## Beschreibung

### Gebiet der Erfindung

Die Erfindung betrifft ein medizinisches Instrument für die minimal-invasive Chirurgie, insbesondere ein medizinisches Instrument, bevorzugt ein Resektoskop, zum Abtragen von Gewebe mittels einer HF-Elektrode mit der Funktion einer kontrollierten Winkelausrichtung des distalen Instrumentenbereichs.

### Hintergrund der Erfindung

Heutzutage sind minimal-invasive Eingriffe aus der modernen Medizin nicht mehr wegzudenken. Für minimal-invasive Eingriffe an der Harnblase oder Prostata findet die sog. transurethrale Resektion (TUR) als urologische oder auch gynäkologische Operationstechnik zum Abtragen von erkranktem Gewebe Anwendung. Die Operation erfolgt endoskopisch durch die Harnröhre ohne äußeren Schnitt. Dabei wird in die Harnblase eine HF-Elektrode eingeführt, an die ein hochfrequenter Wechselstrom angelegt wird, um schädliches oder beschädigtes Gewebe abzutragen.

Herkömmlich verwendete Resektoskope weisen mindestens einen hohlen Schaft auf und sind steif ausgebildet, um diese besser platzierbar zu machen und gleichzeitig eine gute Führung für die HF-Elektrode bereitzustellen. Bei Blasenresektionen, bei denen u.a. schädliches Gewebe aus der Blase entfernt wird, kann sich das abzutragende Gewebe im Inneren der Blase und genauer gesagt seitlich entfernt von dem Harnkanal befinden, also hinter dem Blasenhals. In diesem Fall kann das Resektoskop zwar durch den Harnkanal eingeführt werden, jedoch nicht direkt an den seitlich zu der Einführachse gelegenen Operationsort geführt werden. Dies ist allenfalls durch ein traumatisches Hebeln oder Verkippen des Resektoskops bedingt möglich.

DE 2006 039 696 A1 offenbart eine Vorrichtung zur Resektion und / oder Ablation von organischem Gewebe mittels HF-Strömen. Dabei umfasst die Vorrichtung eine HF-Schlinge und einen entsprechenden schaftförmigen Träger. Die Schlinge kann mit HF-Strömen beaufschlagt werden, sodass mit einer keilförmigen HF-Schneide geschnitten werden kann, um eine elektrochemische HF-Operation durchzuführen. Jedoch ist die HF-Schneide nur entlang einer Mittellinie des Instruments anwendbar. Ein Abknicken oder Abwinkeln der HF-Schneide ist nicht möglich.

EP 2 298 204 B1 der Anmelderin offenbart ebenfalls ein medizinisches Instrument zur bipolaren Elektrochirurgie, das einen (steifen) Außenschaft umfasst, an dessen distalem Ende eine Schneide angeordnet ist, die mit HF-Strömen beaufschlagt werden kann und gleichzeitig gegen den Außenschaft isoliert ist. Auch mit dieser Vorrichtung ist jedoch nur ein Schneiden entlang der Mittellinie des Schafts möglich. Ein Abwinkeln der HF-Schneide ist nicht möglich.

DE 10354830A1 offenbart eine medizinische Hochfrequenz-Schneidevorrichtung mit einem hohlen Schaft, einer gegen diesen elektrisch isolierten HF-Schlinge am distalen Ende des Schafts, die teilweise axial über ihn hinausragt, wobei die HF- Schlinge durch axiales Verschieben relativ zu dem Schaft zu diesem abgewinkelt werden kann, so dass die Schlinge über einen Querschnitt des Schafts hinausragt. Dabei ist eine definierte Winkelausrichtung der HF-Elektrode möglich, jedoch keine veränderbare Winkelausrichtung.

US5976129A offenbart eine endoskopische Hochfrequenz-Schneidevorrichtung mit einem hohlen Schaft, einer gegen diesen elektrisch isolierten HF-Schlinge am distalen Ende des Schafts, die teilweise axial über den Schaft und auch radial über seinen Querschnitt hinausragt, wobei der Winkel, in dem die HF- Schlinge in Gewebe eindringt, anzupassen sei - allerdings ohne Lehre, wie dies erfolgt.

### Zusammenfassung der Erfindung

Es ist daher eine Aufgabe der vorliegenden Erfindung, ein medizinisches Instrument zum minimal-invasiven Abtragen von Gewebe mittels einer HF-Elektrode bereitzustellen, das noch flexibler eingesetzt werden kann, um Gewebe insbesondere auch an herkömmlich schwer zugänglichen Bereichen gezielt abzutragen, insbesondere bei Anwendungen in der transurethralen Resektion.

Diese Aufgabe wird erfindungsgemäß durch das medizinische Instrument nach Anspruch 1 gelöst. Weitere vorteilhafte Ausführungsformen ergeben sich durch die Unteransprüche.

Gemäß der vorliegenden Erfindung wird ein medizinisches Instrument zum minimalinvasiven Abtragen von Gewebe mittels einer HF-Elektrode bereitgestellt, das einen hohlen Schaft aufweist, wobei die HF-Elektrode gegen den hohlen Schaft elektrisch isoliert ist, an einem distalen Ende von diesem angeordnet ist und zum Abtragen von Gewebe zumindest teilweise axial über den hohlen Schaft hinausragt. Erfindungsgemäß kann die Winkelausrichtung der HF-Elektrode relativ zu dem hohlen Schaft kontrolliert in eine Arbeitsstellung verstellt werden, in der die HF-Elektrode, in einer Vorderansicht auf den hohlen Schaft betrachtet, zum Abtragen von Gewebe radial über einen Außenumfang des hohlen Schafts hinausragt.

Aufgrund dieser Winkelverstellung kann der Schaft des Instruments ohne die Gefahr einer Beschädigung der HF-Elektrode oder von Gewebe durch eine Öffnung in den Körper eines Patienten eingeführt werden, insbesondere durch eine Harnröhre, und im Operationsbereich angeordnet werden. Anschließend kann die Winkelstellung der HF-Elektrode geeignet verstellt werden, sodass dann Gewebebereiche beabstandet zur Einführachse zum Abtragen von Gewebe ohne weiteres erreichbar sind.

Gemäß der vorliegenden Erfindung weist die HF-Elektrode ferner einen elektrischen Isolationskanal und eine HF-Schlinge auf, wobei der Isolationskanal die HF-Elektrode gegen den hohlen Schaft elektrisch isoliert und zum Abtragen von Gewebe axial über das distale Ende des Schafts hinausragt, und wobei ein distales Ende der HF-Schlinge zum Abtragen von Gewebe axial über das distale Ende des Isolationskanals hinausragt.

Gemäß der vorliegenden Erfindung ist das distale Ende der HF-Schlinge von einer Abtragekante ausgebildet, die über eine symmetrisch gekrümmte Abtragekante in einen Verbindungsabschnitt zur Beaufschlagung der HF-Schlinge mit einer Hochfrequenzspannung übergeht, wobei die Winkelausrichtung des Verbindungsabschnitts relativ zu einer Mittellinie des hohlen Schafts kontrolliert verstellbar ist, um die Winkelausrichtung der HF-Schlinge relativ zu dem hohlen Schaft kontrolliert zu verstellen.

Das distale Ende der HF-Schlinge ragt dabei in einer Einführstellung insbesondere hakenförmig von Zuleitungen der HF-Elektrode ab, insbesondere unter einem Winkel von 60 bis 180°, beispielsweise im Bereich von oder genau 90 Grad. Bereits kleine Winkelverstellungen der HF-Elektrode können dann in einer vergleichsweise großen Änderung des Abstands des distalen Endes der HF-Schlinge zur Mittellinie des Schafts des medizinischen Instruments resultieren.

Gemäß einer weiteren Ausführungsform besteht der Verbindungsabschnitt aus einem flexiblen oder elastischen, elektrisch leitenden Material. Die Winkelausrichtung der HF-Schlinge relativ zu dem hohlen Schaft kann somit durch Biegen oder Abknicken des Verbindungsabschnitts in vorteilhaft einfacher Weise kontrolliert verstellt werden. Hierzu können insbesondere die Materialstärke bzw. der Durchmesser des vorgenannten Verbindungsabschnitts, bei ansonsten gleichem Material, im Vergleich zu den übrigen Abschnitten der HF-Elektrode reduziert werden.

Gemäß einer weiteren Ausführungsform ist die HF-Elektrode in die Arbeitsstellung bevorzugt elastisch vorgespannt. Die HF-Elektrode ist damit belastbarer und gibt bei Berührung mit den abzutragenden Gewebeabschnitten weniger nach, was in einer höheren Positionierungsgenauigkeit der HF-Elektrode resultiert. In der Arbeitsstellung ist dabei ein distales Ende des Verbindungsabschnitts relativ zu einem proximalen Ende des Verbindungsabschnitts abgebogen oder abgeknickt, um mit der Mittellinie des hohlen Schafts einen geeigneten spitzen Winkel einzuschließen.

Gemäß einer weiteren Ausführungsform ist die HF-Elektrode gegen den hohlen Schaft längsverschieblich geführt, wobei durch Verstellen der HF-Elektrode hin zum proximalen Ende des hohlen Schafts die HF-Elektrode in eine Einführstellung verstellbar ist, in der das distale Ende des Verbindungsabschnitts mit dem proximalen Ende des Verbindungsabschnitts im Wesentlichen fluchtet und die HF-Schlinge bevorzugt nicht über ein von Innenseiten des hohlen Schafts ausgebildetes Innenprofil hinausragt, um die Gefahr von Schädigungen von Gewebe oder der HF-Elektrode zu reduzieren. Bevorzugt wird bei einer axialen Verstellung der HF-Elektrode automatisch auch deren Winkelstellung verändert. Bevorzugter wird mit einer zunehmenden axialen Verstellung der Neigungswinkel der HF-Elektrode relativ zur Mittellinie des Schafts automatisch zunehmend verändert. Durch Wahl der axialen Verstellung kann somit auch die Winkelstellung der HF-Elektrode präzise eingestellt werden, was eine vorteilhaft einfache Positionierung ermöglicht.

Gemäß einer weiteren Ausführungsform ist hierzu der Verbindungsabschnitt relativ zu dem elektrischen Isolationskanal oder ist der elektrische Isolationskanal relativ zu dem hohlen Schaft längsverschieblich geführt, was den Aufbau des medizinischen Instruments vorteilhaft einfach machen kann.

Gemäß einer weiteren Ausführungsform ist der Verbindungsabschnitt in einem Überrohr längsverschieblich geführt, das relativ zu der Mittellinie des hohlen Schafts bogenförmig gekrümmt ist oder durch geeignete Verstellung bogenförmig gekrümmt werden kann, sodass die Position und Winkelausrichtung der HF-Schlinge relativ zu dem hohlen Schaft durch eine einfache Längsverstellung der HF-Elektrode kontrolliert verstellbar ist, um den Verbindungsabschnitt abzubiegen oder abzuknicken.

Gemäß einer weiteren Ausführungsform ist das Überrohr unmittelbar als Abschnitt des Isolationskanals ausgebildet.

Gemäß einer weiteren Ausführungsform ist das Überrohr alternativ als hohle Zug- oder Druckstange ausgebildet, die mit der HF-Elektrode gekoppelt ist. Durch mechanische Verstellung der Zug- oder Druckstange lässt sich so in vorteilhaft einfacher Weise eine präzise Positionierbarkeit der HF-Elektrode erzielen.

Gemäß einer weiteren Ausführungsform ist das Überrohr alternativ aus einem Memory-Material ausgebildet, dessen Ruhestellung in Bezug zu der Mittellinie des hohlen Schafts gestreckt oder abgewinkelt ist, wobei die Orientierung eines distalen Endes des Überrohrs relativ zu der Mittellinie des hohlen Schafts verstellbar ist, insbesondere durch Anlegen eines elektrischen Stroms oder eine Temperaturänderung. Durch Ändern von einfachen physikalischen Parametern kann so die HF-Elektrode präzise und reproduzierbar positioniert werden.

Gemäß der vorliegenden Erfindung ist am distalen Ende des hohlen Schafts ein Umlenkelement vorgesehen, an dem der Verbindungsabschnitt oder der elektrische Isolationskanal unmittelbar anliegt, wobei das Umlenkelement dergestalt ausgebildet ist, dass durch Längsverschiebung der HF-Elektrode gegen den hohlen Schaft ein zunehmendes Abbiegen oder Abknicken des Verbindungsabschnitts oder des elektrischen Isolationskanals bewirkt wird. Hierzu ist das Umlenkelement zweckmäßig weniger weit beabstandet von der Mittellinie des hohlen Schafts angeordnet als die Zuleitungen der HF-Elektrode. Mit zunehmender distaler Verstellung der HF-Elektrode entfernt sich somit aufgrund einer Anlage der Zuleitungen an dem Umlenkelement die HF-Elektrode immer stärker von der Mittellinie, insbesondere durch Abbiegen oder Abknicken des vorgenannten Verbindungsabschnitts.

Gemäß einer weiteren Ausführungsform ist weiterhin ein verstellbares Abwinklungselement vorgesehen, wobei durch Verstellen des Abwinklungselements in Anlage mit einem Abschnitt der HF-Elektrode oder des elektrischen Isolationskanals die Winkelausrichtung der HF-Schlinge relativ zu dem hohlen Schaft kontrolliert verstellbar ist. Das Abwinklungselement kann hierzu über einen Zug, ein Gestänge oder dergleichen mechanisch verstellbar sein.

Gemäß einer weiteren Ausführungsform weist das Abwinklungselement ein piezoelektrisches Element oder ein thermisch verstellbares Element auf, sodass die Winkelstellung der HF-Elektrode durch einfaches Ändern von physikalischen Parametern präzise verstellt werden kann.

Gemäß einer weiteren Ausführungsform weist die HF-Elektrode Gelenke auf, die an dem Verbindungsabschnitt angeordnet sind, sodass die Winkelausrichtung der HF-Schlinge relativ zu dem hohlen Schaft durch Verstellen der Gelenke, insbesondere durch Abknicken der Gelenke, kontrolliert verstellt werden kann. Dies kann insgesamt eine höhere Steifigkeit der HF-Elektrode und ein geringeres Nachgeben derselben bei Berührung mit abzutragendem Gewebe ermöglichen, insbesondere weil Verbindungs- oder Zuleitungsabschnitte der HF-Elektrode nicht mit geringerer Materialstärke ausgebildet zu sein brauchen. Hierzu können den Gelenken insbesondere elastische Rückstellmittel zugeordnet sein, um die HF-Schlinge in eine gestreckte oder abgewinkelte Ruhestellung elastisch vorzuspannen.

Gemäß einer weiteren Ausführungsform ist eine vorteilhaft einfache, präzise und reproduzierbare Verstellung der Winkelausrichtung der HF-Elektrode dadurch realisiert, dass die HF-Elektrode zumindest abschnittsweise aus einem Memorymetall ausgebildet ist, das ausgelegt ist, sodass die Winkelausrichtung der HF-Schlinge relativ zu dem hohlen Schaft kontrolliert verstellbar ist, insbesondere durch Anlegen eines elektrischen Stroms oder eine Temperaturänderung.

Gemäß einer weiteren Ausführungsform weist der hohle Schaft einen hohlen Außenschaft und einen darin aufgenommenen Innenschaft auf, wobei der Innenschaft in dem hohlen Außenschaft längsverschieblich geführt ist und wobei die HF-Elektrode vollständig in den hohlen Außenschaft zurückgefahren werden kann. Durch die Längsverschieblichkeit des Innenschafts relativ zu dem Außenschaft kann so der Verstellbereich der HF-Elektrode weiter vergrößert werden.

Gemäß einer weiteren Ausführungsform kann ein Neigungswinkel der HF-Elektrode relativ zu der Mittellinie des hohlen Schafts um einen Winkel von bis zu 90° verstellt werden. Bevorzugter liegt dieser Verstellwinkel in einem Bereich von 50° bis 75° liegt, was dem typischen Öffnungswinkel der menschlichen Harnblase im Bereich des Blasenhalses entspricht. Der maximale (minimale) Verstellwinkel ist zweckmäßig geringfügig größer als der typische maximale (minimale) Öffnungswinkel der menschlichen Harnblase im Bereich des Blasenhalses. Für eine zuverlässige Begrenzung dieses Winkel-Verstellbereichs können mechanische Winkelanschläge oder dergleichen vorgesehen sein.

### Figurenübersicht

Nachfolgend werden bevorzugte Ausführungsformen nach der Erfindung anhand der beigefügten Zeichnungen näher beschrieben, woraus sich weitere Merkmale, Vorteile und zu lösende Aufgaben ergeben werden. Es zeigen:
- Fig. 1: eine schematische Perspektivansicht des gesamten medizinischen Instrumentes nach einer bevorzugen Ausführungsform der vorliegenden Erfindung;
- Fig. 2: eine vergrößerte Perspektivansicht des distalen Bereichs des medizinischen Instrumentes nach einer bevorzugen Ausführungsform der vorliegenden Erfindung, mit einer in ihrer Winkelausrichtung verstellbaren HF-Elektrode in einem (noch) nicht abgewinkelten Zustand;
- Fig. 3a: eine schematische Perspektivansicht des distalen Bereichs des medizinischen Instrumentes nach der Fig. 2 in einer abgewinkelten Stellung bzw. Arbeitsstellung der HF-Elektrode;
- Fig. 3b: eine schematische Perspektivansicht des distalen Bereichs des medizinischen Instrumentes nach einer weiteren Ausführungsform der vorliegenden Erfindung, mit einer HF-Schlinge, deren Winkelstellung über Gelenke kontrolliert verstellbar ist;
- Fig. 3c: in einer Seitenansicht den distalen Bereich nach der Fig. 3b in einer abgewinkelten Stellung der HF-Schlinge;
- Fig. 4: eine Seitenansicht des distalen Bereichs des medizinischen Instrumentes nach einer weiteren Ausführungsform der vorliegenden Erfindung, wobei die Winkelstellung der HF-Elektrode mittels eines Abwinklungshebels verstellbar ist;
- Fig. 5a: eine schematische Perspektivansicht des distalen Bereichs des medizinischen Instrumentes nach einer weiteren Ausführungsform der vorliegenden Erfindung, mit einer distal angeordneten HF-Elektrode, deren Winkelstellung durch Biegen bzw. Abwinkeln eines elektrischen Isolationskanals verstellbar ist;
- Fig. 5b: eine schematische Perspektivansicht des distalen Bereichs des medizinischen Instrumentes nach einer weiteren Ausführungsform der vorliegenden Erfindung, mit einer distal angeordneten HF-Elektrode, deren Winkelstellung durch Biegen bzw. Abwinkeln eines distalen Abschnitts eines Innenschafts verstellbar ist; und
- Fig. 6: die geometrischen Verhältnisse bei einem minimal-invasiven Eingriff an der Harnblase eines menschlichen Patienten, zur weiteren Veranschaulichung der Vorteile gemäß der vorliegenden Erfindung.

In den Figuren bezeichnen identische Bezugszeichen identische oder im Wesentlichen gleichwirkende Elemente oder Elementgruppen.

### Ausführliche Beschreibung von Ausführungsbeispielen

Bezüglich der Begriffserklärung, wird an dieser Stelle darauf verwiesen, dass "distal" im Sinne von "zu dem Operationsort hin" bzw. "von dem Operateur weg" zu verstehen ist und synonym mit "vorne" verwendet wird. Die Bezeichnung "proximal" ist konträr hierzu, d.h. im Sinne von "zu dem Operateur hin" bzw. "von dem Operationsort weg" zu verstehen und wird synonym mit "hinten" verwendet.

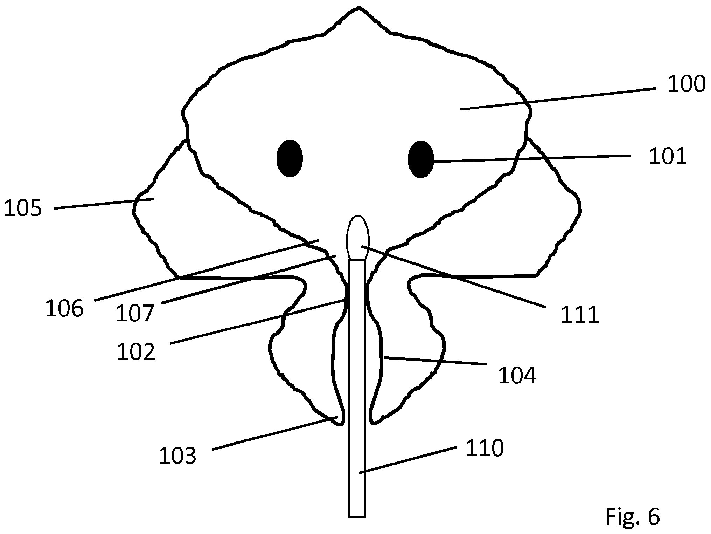

Die Fig. 6 fasst zunächst die geometrischen Verhältnisse bei minimal-invasiven Eingriffen an der als Hohlorgan ausgebildeten menschlichen Harnblase 100 zusammen. Die Harnleiter münden paarig in den Bereichen 101 in die Harnblase 100. Das Trigonum vesicae 106 ist die dreieckige Fläche, die von Eingängen der beiden Harnleiter und vom Ausgang der Harnröhre gebildet wird. Diese innerste Schicht der Harnblase wird auch Mucosa 105 genannt. Der Schaft 110 eines medizinischen Instruments kann über die Harnröhre und über den Bereich des äußeren Schließmuskels 103 sowie des inneren Schließmuskels 102 in die Harnblase 100 eingeführt werden und passiert dabei auch den Bereich der Prostata 104. Mit einer üblicherweise am distalen Ende des Schafts 110 befindlichen HF-Elektrode 111 sind deshalb herkömmlich nur Bereiche nahe einer Einführachse zugänglich, die im Wesentlichen durch die beiden Schließmuskelbereiche 102, 103 bzw. die Harnröhre festgelegt ist. Wenn allerdings ein Tumor oder geschädigtes Gewebe entfernt von dieser Einführachse gelegen ist, sind minimal-invasive Eingriffe herkömmlich nur eingeschränkt möglich. Ein Verkippen des Schafts 110 auch nur um kleine Winkel führt ohne weiteres zu einer Schädigung von gesundem Gewebe und ist außerdem äußerst traumatisch.

Wie nachfolgend ausgeführt, ermöglicht ein medizinisches Instrument gemäß der vorliegenden Erfindung minimal-invasive Eingriffe gerade auch abseits der vorgenannten Einführachse, insbesondere im Bereich der Prostata 104 und im Bereich des Blasenhalses 107, der üblicherweise einen Öffnungswinkel im Bereich von etwa 50° bis etwa 75° hat. Wie man der Fig. 6 entnehmen kann, sind Gewebeabschnitte auf der Innenwand der Harnblase im Bereich des Blasenhalses 107 nicht allzu weit seitlich beabstandet zur vorgenannten Einführachse gelegen. Um solche Gewebeabschnitte gerade in diesem Bereich gezielt mittels einer HF-Elektrode 111 abzutragen, kann die Winkelausrichtung der HF-Elektrode 111 erfindungsgemäß relativ zu dem Schaft 110 kontrolliert in eine Arbeitsstellung verstellt werden, in der die HF-Elektrode 111, in einer Vorderansicht auf den Schaft 110 betrachtet, radial über einen Außenumfang des Schafts 110 hinausragt. In dieser Arbeitsstellung kann durch Längsverstellung des Schafts 110, also durch Verschieben längs der vorgenannten Einführachse, stets eine Stellung gefunden werden, in der das distale Ende der HF-Elektrode 111 abzutragende Gewebeabschnitte im Bereich des Blasenhalses 107 in ausreichendem Maße erreicht bzw. berührt. Um Gewebeabschnitte in anderen Bereichen des Blasenhalses 107 zu erreichen, braucht der Schaft 110 einfach nur axial entlang der vorgenannten Einführachse verstellt zu werden und/oder der Neigungswinkel der HF-Elektrode 111 relativ zu dem Schaft 110 bzw. der vorgenannten Einführachse weiter verstellt zu werden. Mit anderen Worten: durch geeignet tiefes Einführen des Schafts 110 und/oder Verstellung der Winkelausrichtung der HF-Elektrode sind erfindungsgemäß sämtliche Gewebeabschnitte im Bereich des Blasenhalses 107 erreichbar.

Wie man der Fig. 6 ohne weiteres entnehmen kann, gilt dies in entsprechender Weise auch für Gewebeabschnitte im Bereich der Prostata 104, die abseits der vorgenannten Einführachse gelegen sind.

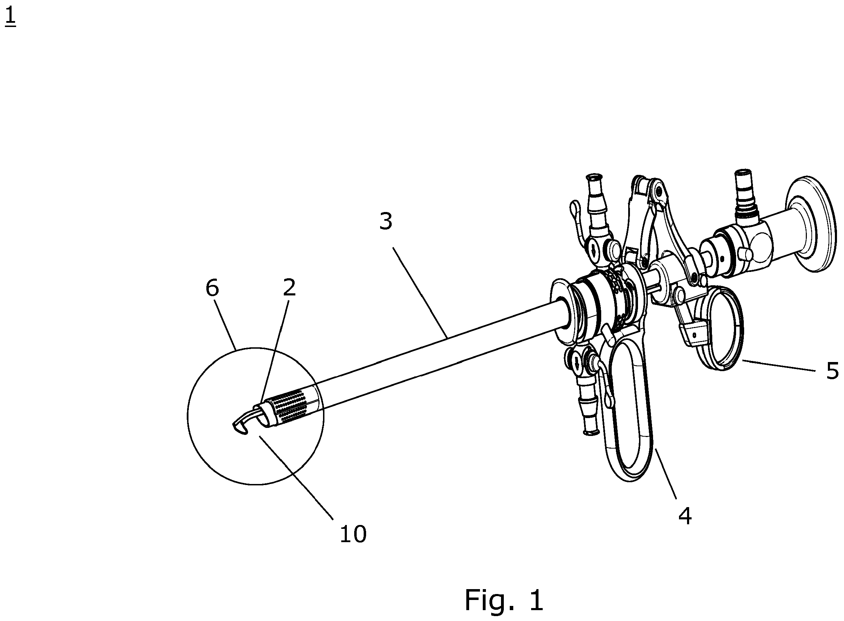

Fig. 1 zeigt ein medizinisches Instrument 1, das als Basis für die Ausführungsformen der vorliegenden Erfindung zum Abtragen von Gewebe, insbesondere von Gewebe im Bereich des Blasenhalses oder der Prostata, durch einen minimal-invasiven chirurgischen Eingriff dient. Dabei weist das medizinische Instrument im Wesentlichen drei Abschnitte auf: einen vorderen oder auch distalen Abschnitt 6, einen sich an einem hinteren oder proximalen Ende befindlichen Bedienabschnitt 5 und einen durch den Schaft 3 ausgebildeten Abschnitt, der sich zwischen den zuvor genannten Abschnitten erstreckt und jeweils mit diesen verbunden ist.

Das distale Ende 6 befindet sich am vorderen Ende des Schafts 3 und umfasst insbesondere eine vordere Öffnung, aus der eine HF-Elektrode 10 abragt, die zum Abtragen von Gewebe durch Anlegen eines hochfrequenten Wechselstroms in der üblichen Weise dient.

Der Schaft 3 weist eine im Wesentlichen zylindrische Form auf und hat eine angemessene Länge und Dimension, um in einen menschlichen Körper eingeführt zu werden. In einer alternativen Gestaltung kann der Schaft auch eine ovale Form oder andere Rohrform aufweisen. Mit dem Schaft 3 können in einem minimal-invasiven chirurgischen Eingriff Operationsinstrumente an den Ort einer Operation in den menschlichen Körper eingeführt werden.

Der Bedienabschnitt weist im Wesentlichen zwei Teilabschnitte auf, nämlich einen Platziergriffabschnitt und einen Operationsgriffabschnitt, die längsbeweglich miteinander über eine Übertragungswelle zum Übertragen einer Kraft und über ein Positionierungs-Scharnier zum Positionieren und zum Führen des Schafts verbunden sind.

Am distalen Ende des Bedienabschnitts ist insbesondere ein Platziergriff 4 angeordnet, der starr mit dem äußeren Teil des Schafts 3 verbunden sein kann. Dadurch kann der Platziergriff 4 benutzt werden, um das medizinische Instrument 1 in den menschlichen Körper einzuführen und am Operationsort zu platzieren.

Am Operationsgriffabschnitt am proximalen Ende des Bedienabschnitts sind ein Okular und ein Operationsgriff 5 angeordnet. Der Operateur (d.h. der Chirurg) ist nun in der Lage, seinen Daumen durch den Operationsgriff 5 zu führen und die übrigen Finger einer Hand durch den Platziergriff 4, um durch Öffnen und Schließen der Hand eine Vorwärts- und Rückwärtsbewegung eines Innenschafts 2 relativ zu dem hohlen Außenschaft des Schaftes 3 auszuführen, sodass der Operateur mit nur einer Hand operieren kann.

Mithilfe des Operationsgriffes 5 können Teile des medizinischen Instruments 1 zusätzlich gedreht werden. Insbesondere ist das Drehen eines Innenschafts 2 relativ zu einem Außenschaft des Schaftes 3 möglich.

Folglich ist das medizinische Instrument 1 nach dieser Ausführungsform also ein Resektoskop mit einem Innenschaft 2, an dessen distalem Ende eine HF-Elektrode 10 zum Abtragen von Gewebe durch Anlegen von hochfrequenten Wechselströmen angeordnet ist.

An dieser Stelle sei darauf verwiesen, dass nicht alle Bestandteile des obigen Ausführungsbeispiels für die vorliegende Erfindung notwendig sind. Insbesondere kann der proximale Abschnitt des medizinischen Instrumentes 1 auch anders, als in Fig. 1 dargestellt, ausgebildet sein. Obwohl in dem Ausführungsbeispiel zwei Schäfte, nämlich ein Innenschaft 2 und ein Außenschaft 3 dargestellt werden, ist für die Grundausführung der vorliegenden Erfindung lediglich ein Schaft notwendig. Erfindungsgemäß kann die Winkelausrichtung der HF-Elektrode 10 relativ zu dem Innenschaft 2 bzw. dem Außenschaft 3 kontrolliert verstellt werden, wie nachfolgend ausgeführt.

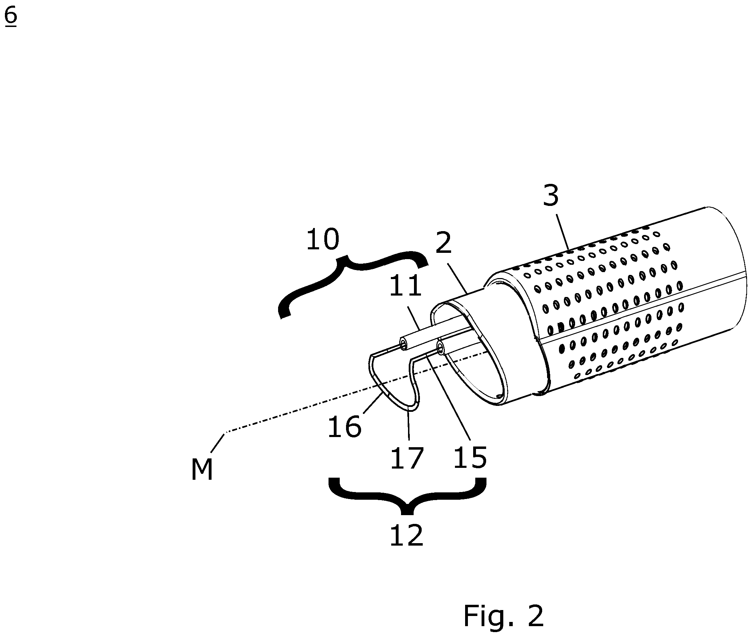

Fig. 2 zeigt eine detaillierte Ansicht des distalen Abschnitts 6 des medizinischen Instrumentes 1 nach der Fig. 1. Im Detail zeigt Fig. 2 die distalen Abschnitte des Innenschafts 2 und des Außenschafts 3, wobei das distale Ende des Innenschafts 2 axial über die Öffnung des Außenschafts 3 hinausragt. Wie schon zuvor erwähnt, kann der Innenschaft 2 längsverschieblich in dem Außenschaft 3 geführt sein. Gemäß Fig. 2 sind der Außenschaft 3 und der Innenschaft 2 jeweils parallel zur Mittellinie M ausgerichtet, die im Folgenden als Referenzlinie für die Winkelausrichtung der HF-Elektrode 10 verwendet wird.

Der Außenschaft 3 weist an seinem distalen Ende eine Vielzahl von Mündungslöchern auf, die auf der Außenwand des Außenschafts 3 verteilt angeordnet sind. Durch die Mündungslöcher kann eine Spülflüssigkeit rückgeführt werden.

Die HF-Elektrode 10 weist eine HF-Schlinge 12 und einen Isolationskanal 11 auf, wobei die HF-Schlinge 12 in dem Isolationskanal 11 geführt ist, um gegenüber dem Innenschaft 2 elektrisch isoliert zu sein. Der Isolationskanal 11 besteht aus einem elektrisch isolierenden Material und isoliert die HF-Schlinge 12 und deren elektrische Zuführungen elektrisch gegen den Innenschaft 2 und Außenschaft 3. Zum Abtragen von Gewebe ragt der Isolationskanal 11 zumindest geringfügig axial über das distale Ende des Innenschafts 2 hinaus. Isolationskanal 11 und HF-Schlinge 12 können dabei starr relativ zu dem Innenschaft 2 gelagert sein, können jedoch bevorzugt gegenüber dem Innenschaft 2 auch axial verstellt werden, um die HF-Schlinge 12 in eine Arbeitsstellung zu überführen, wie nachfolgend näher ausgeführt.

Gemäß der Fig. 2 ragt die HF-Schlinge 12 axial über die distale Öffnung des Isolationskanals 11 hinaus, und weist drei Teilabschnitte auf: einen Verbindungsabschnitt 15, eine seitliche Abtragekante 16 und eine symmetrisch gekrümmte Abtragekante 17.

Gemäß der Fig. 2 ist am distalen Ende der HF-Schlinge eine symmetrisch gekrümmte Abtragekante 17 ausgebildet, die seitlich symmetrisch abgerundet ist. Durch diese Anordnung kann eine aufgebrachte Kraft in einem kleinen vorderen Bereich der symmetrisch gekrümmten Abtragekante 17 konzentriert werden. Insbesondere kann die symmetrisch gekrümmte Abtragekante 17 gleichmäßig symmetrisch gekrümmt ausgebildet sein. Die Abtragekante 17 erstreckt sich dabei zweckmäßig senkrecht zur Mittellinie M, kann sich jedoch gemäß weiteren Ausführungsformen grundsätzlich auch unter einem spitzen Winkel geneigt zu dieser Mittellinie M erstrecken. Der Abtragekante 17 schließt sich seitlich eine seitliche Abtragekante 16 an, die über einen Verbindungsabschnitt 15 mit Zuführleitungen verbunden sind, die in dem Isolationskanal 11 verlaufen und der Einspeisung eines hochfrequenten Wechselstroms dienen.

In der in der Fig. 2 gezeigten Einführstellung ragt die HF-Schlinge 12 dabei während des Einführens des distalen Endes des Schafts 2, 3 bevorzugt radial nicht über das Außenprofil des Außenschafts 3 hinaus, sodass die HF-Schlinge 12 in der Einführstellung keine Gewebeschädigungen hervorrufen kann. Zu diesem Zweck kann die HF-Schlinge 12 auch axial zum distalen Ende des Schafts 2, 3 zurückgefahren werden oder gar in den Innenraum des hohlen Innenschafts 2 vollständig zurückgefahren werden.

Wie nachfolgend ausgeführt, kann die Winkelausrichtung der HF-Schlinge 12 relativ zu der Mittellinie M kontrolliert verstellt werden, sodass diese in eine Arbeitsstellung überführt werden kann, in der die HF-Schlinge radial über das Außenprofil des Außenschafts 3 hinausragt. Hierzu besteht der Verbindungsabschnitt 15 aus einem flexiblen oder elastischen, elektrisch leitenden Material, sodass die Winkelausrichtung der HF-Schlinge 12 relativ zu der Mittellinie M durch Biegen oder Abknicken des Verbindungsabschnitts 15 kontrolliert verstellt werden kann. Die restlichen Abschnitte der HF-Elektrode 10, wie beispielsweise die Abtragekante 17, können dabei durchaus im Vergleich zum Verbindungsabschnitt 15 weniger flexibel bzw. elastisch ausgebildet sein, beispielsweise bedingt durch eine höhere Materialstärke des elektrischen Leiters in diesen restlichen Abschnitten.

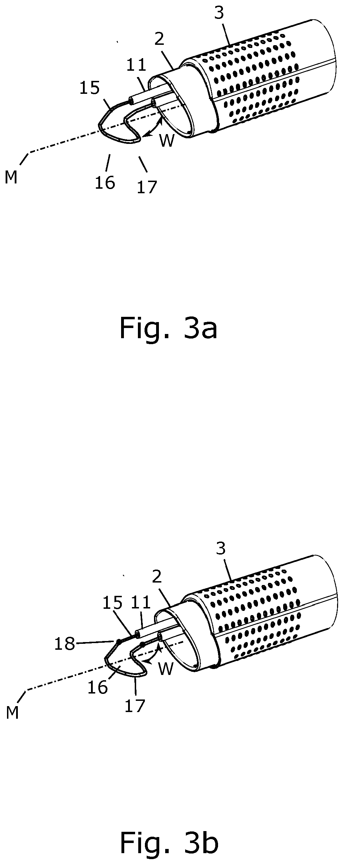

Fig. 3a zeigt eine detaillierte Ansicht des distalen Abschnitts 6 des medizinischen Instrumentes 1 gemäß einer ersten bevorzugten Ausführungsform. Bei dieser Ausführungsform kann die Winkelausrichtung der HF-Schlinge relativ zu der Mittellinie M durch Biegen des Verbindungsabschnitts 15 kontrolliert verstellt werden, wie durch den Doppelpfeil angedeutet. Dadurch kann die Größe des Winkels W zwischen der HF-Schlinge und der Mittellinie M verändert werden.

Hierzu ist die HF-Schlinge in die in der Fig. 3a gezeigte Arbeitsstellung vorgespannt, bevorzugt elastisch vorgespannt, was durch geeignete Formgebung und Materialwahl der HF-Schlinge ohne weiteres erzielt werden kann. Durch ein Zuggestänge, das mit der HF-Schlinge gekoppelt ist, kann nun die HF-Schlinge axial hin zum distalen Ende des Innenschafts 2 verstellt werden. Dadurch gerät schließlich der Isolationskanal 11 in Anlage mit der Innenwand des Innenschafts 2 oder mit am distalen Ende des Innenschafts 2 vorgesehenen Führungselementen, wodurch ein Zurückbiegen der HF-Schlinge zurück in die Einführstellung gemäß der Fig. 2 bewirkt wird. Dieses Zurückstellen der HF-Schlinge von der Arbeitsstellung in die Ruhestellung kann dabei kontinuierlich erfolgen, d.h. der Neigungswinkel der HF-Schlinge relativ zu der Mittellinie M nimmt immer mehr ab, je weiter die HF-Schlinge zurück zum distalen Ende des Innenschafts eingefahren wird. Gemäß einer weiteren Ausführungsform kann das Material der HF-Schlinge jedenfalls im Bereich des Verbindungsabschnitts 15 auch eine Art bistabiles Verhalten bedingen, sodass sich der Neigungswinkel der HF-Schlinge relativ zu der Mittellinie M beim Einfahren der HF-Schlinge hin zum distalen Ende des Innenschafts 2 zunächst nicht wesentlich ändert, jedoch bei Überschreiten eines vorbestimmten Bereichs die HF-Schlinge schließlich in die nicht abgewinkelte Ruhestellung gemäß der Fig. 2 schnappt, oder so dass sich der Neigungswinkel der HF-Schlinge relativ zu der Mittellinie M beim Herausfahren der HF-Schlinge weg vom distalen Ende des Innenschafts 2 zunächst nicht wesentlich ändert, jedoch bei Überschreiten eines vorbestimmten Bereichs die HF-Schlinge schließlich in die abgewinkelte Arbeitsstellung gemäß der Fig. 3a schnappt.

Zu diesem Zweck kann die HF-Schlinge 12 oder zumindest deren Verbindungsabschnitt 15 auch aus einem (elektrisch leitenden) Memorymetall bestehen, insbesondere einer elektrisch leitenden Formgedächtnislegierung. In einem solchen Fall kann der Neigungswinkel der HF-Schlinge relativ zu der Mittellinie auch durch geeignete Änderung eines Stroms oder bevorzugt der Temperatur des Memorymetalls kontrolliert verändert werden.

Durch Einziehen bzw. Ausfahren der HF-Schlinge 12 in den bzw. aus dem Innenschaft 2 lässt sich ein Winkel W zwischen der Mittellinie M und der HF-Schlinge 12 kontrolliert verstellen.

Fig. 3b zeigt die detaillierte Ansicht des distalen Abschnitts 6 des medizinischen Instrumentes 1 nach einer weiteren Ausführungsform, bei der die Winkelausrichtung der HF-Schlinge relativ zu der Mittellinie M über im Bereich der HF-Schlinge vorgesehene Gelenke 18 kontrolliert verstellt werden kann. Zweckmäßig sind die Gelenke 18 im Bereich der Verbindungsabschnitte 15 oder im Übergangsbereich zur seitlichen Abtragekante 16 vorgesehen, wie in der Fig. 3b gezeigt. Die Winkelbeweglichkeit der Gelenke 18 ist dabei zweckmäßig auf den gewünschten Winkelbereich beschränkt, was sich durch Ausgestaltung beispielsweise als Drehscharniere mit geeigneten Winkelanschlägen in einfacher Weise realisieren lässt. Die HF-Schlinge kann somit auf Anschlag in eine Ruhestellung verstellt werden, in der der Verbindungsabschnitt 15 sich parallel zur Mittellinie M erstreckt und die HF-Schlinge bevorzugt nicht über ein von Innenseiten des Innenschafts 2 ausgebildetes Innenprofil oder über ein von Außenseiten des Außenschafts 3 ausgebildetes Außenprofil hinausragt. Weiter kann die HF-Schlinge auf Anschlag in eine geeignete Arbeitsstellung verstellt werden, in der die HF-Schlinge unter einem spitzen Winkel relativ zu der Mittellinie M geneigt ist und radial über das Außenprofil des Außenschafts 3 hinausragt. Die mechanische Verstellung der HF-Schlinge kann hierzu insbesondere mittels eines Zug- oder Druckgestänges erfolgen, das mit der HF-Schlinge gekoppelt ist.

An den Gelenken 18 können Federn angeordnet sein, um die HF-Schlinge 12 elastisch in die Ruhestellung oder Arbeitsstellung vorzuspannen.

Diese Ausführungsform eignet sich insbesondere für eine Kombination mit einem Hebelmechanismus zur kontrollierten Verstellung des Neigungswinkels der HF-Schlinge relativ zu der Mittellinie M, wie nachfolgend ausgeführt, wobei in diesem Fall die Federn dazu ausgelegt sein können, um den Verbindungsabschnitt 15 der HF-Schlinge 12 gerade zu halten.

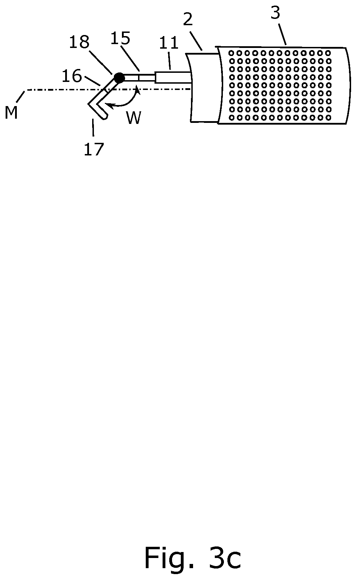

Fig. 3c zeigt in einer Seitenansicht den distalen Bereich nach der Fig. 3b in einer abgewinkelten Arbeitsstellung der HF-Schlinge, in der die HF-Schlinge bevorzugt radial über das Außenprofil des Außenschafts 3 hinausragt.

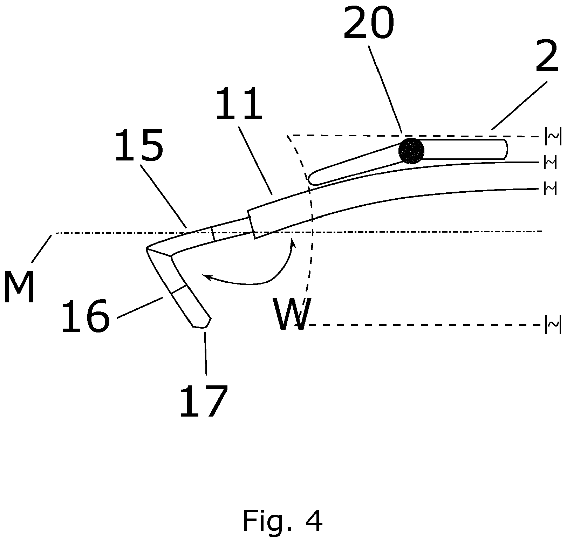

Fig. 4 zeigt eine Seitenansicht des distalen Abschnitts 6 des medizinischen Instrumentes 1 nach einer weiteren Ausführungsform, wobei Einzelheiten des Innenschafts 2 aus Vereinfachungsgründen weggelassen sind. In dieser Ausführungsform ist als Abwinklungselement ein zusätzlicher Abwinklungshebel 20 am distalen Ende des hohlen Innenschafts 2 angeordnet. Bei dieser Ausführungsform kann das distale Ende des Abwinklungshebels 20 um eine Achse rotiert werden, bis dieser schließlich in Anlage mit der Außenseite des Isolationskanals 11 gelangt. Durch weitere Änderung der Winkelstellung des distalen Endes des Abwinklungshebels 20 wird schließlich der Isolationskanal 11 gemeinsam mit den darin geführten Leiterabschnitten der HF-Elektrode weiter gebogen oder abgeknickt, bis schließlich eine Arbeitsstellung erreicht ist, die in der Fig. 4 dargestellt ist. Durch Zurückstellen der Winkelstellung des distalen Endes des Abwinklungshebels 20 kann umgekehrt das distale Ende des Abwinklungshebels 20 von der Außenseite des Isolationskanals 11 wieder gelöst werden, sodass der Isolationskanal 11 gemeinsam mit den darin geführten Leiterabschnitten der HF-Elektrode zurück in eine Ruhestellung verstellt werden können. Die Winkelstellung des Abwinklungshebels 20 kann dabei bevorzugt kontinuierlich verändert werden, wodurch eine sehr feine Veränderlichkeit der Winkelausrichtung der HF-Elektrode relativ zu der Mittellinie M erzielt werden kann. Zum Bedienen des Abwinklungshebels 20 können Bediendrähte oder Leitungen in dem Innenschaft 2 oder Außenschaft 3 geführt sein.

Statt eines mechanischen Abwinklungshebels 20 kann als Abwinklungselement selbstverständlich auch ein piezoelektrisches Element oder ein thermisch verstellbares Element verwendet werden.

Alternativ kann der Abwinklungshebel 20 grundsätzlich auch dazu ausgelegt sein, um nur die Winkelausrichtung der HF-Schlinge 12 zu verstellen, nämlich durch Zusammenwirken nur mit elektrisch leitenden Abschnitten der HF-Schlinge, beispielsweise des elastisch und flexibel ausgelegten Verbindungsabschnitts 15. In einem solchen Fall ist der Abwinklungshebel 20 aus einem elektrisch isolierenden Material ausgebildet, beispielsweise aus einem nicht-leitfähigen Kunststoff, oder zumindest elektrisch isoliert gegen den Innenschaft 2 drehbeweglich gelagert. Oder der Innenschaft 2 ist gegenüber dem Außenschaft (in der Fig. 4 nicht gezeigt) elektrisch isoliert geführt.

Gemäß einer weiteren Ausführungsform kann das Abwinklungselement 20 auch ortsfest und nicht-drehbeweglich am distalen Ende des Innenschafts 2 angeordnet sein. Dabei ist die HF-Elektrode axial verschieblich in dem hohlen Innenschaft 2 gelagert und kann ausreichend weit hinein in den Innenschaft 2 zurückgefahren werden. Wenn die HF-Elektrode axial aus dem Innenschaft 2 herausgefahren wird, gelangt schließlich die HF-Elektrode in Anlage mit dem Abwinklungselement 20, dessen Formgestaltung beim weiteren Herausfahren der HF-Elektrode aus dem Innenschaft 2 dann eine kontrollierte Winkelverstellung der HF-Elektrode bewirkt. Wie in der Fig. 4 schematisch gezeigt, kann das Abwinklungselement 20 beispielsweise bogenförmig gekrümmt ausgebildet sein, in der Art eines bogenförmig gekrümmten Abwinklungsvorsprungs auf dem Innenumfang des hohlen Innenschafts 2, wobei die Zuleitungen der HF-Elektrode auf der Innenseite des Innenschafts 2 radial weiter beabstandet zur Mittellinie M verlaufen als das geometrische Zentrum des Abwinklungselements 20. Beim Herausfahren der HF-Elektrode aus dem Innenschaft 2 gelangen somit zunächst Abschnitte der HF-Elektrode in Anlage mit dem Abwinklungselement 20. Beim Herausfahren der HF-Elektrode aus dem Innenschaft 2 wird die HF-Elektrode zunehmend abgebogen oder abgeknickt, beispielsweise im Bereich des Verbindungsabschnitts 15 oder der in diesem Bereich vorgesehenen Gelenke.

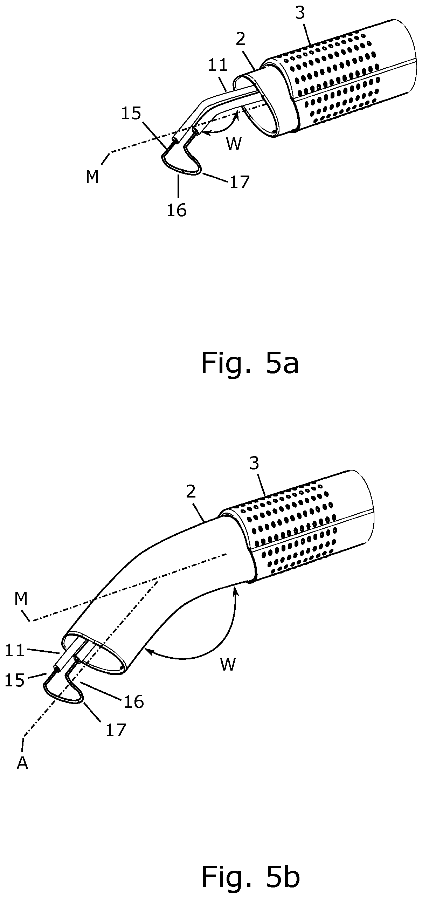

Fig. 5a zeigt die detaillierte Ansicht des distalen Abschnitts 6 des medizinischen Instrumentes 1 nach einer weiteren Ausführungsform. Bei dieser Ausführungsform besteht der Isolationskanal 11 oder zumindest das distale Ende des Isolationskanals 11 aus einem Memory-Material, dessen Ruhestellung in Bezug zu der Mittellinie M des hohlen Innenschafts 2 gestreckt oder abgewinkelt ist. Durch Anlegen einer elektrischen Spannung, geeignete Temperaturänderung oder Änderung weiterer physikalischer Parameter des Memory-Materials kann der Isolationskanal gebogen oder zurück in eine gestreckte Stellung überführt werden, was entsprechend die darin geführten Leiterabschnitte der HF-Elektrode mit verformt. Dadurch kann der Verlauf des Isolationskanals verändert werden und somit die Winkelausrichtung der HF-Elektrode relativ zu der Mittellinie M wie gewünscht kontrolliert verstellt werden.

Fig. 5b zeigt die detaillierte Ansicht des distalen Abschnitts 6 des medizinischen Instrumentes 1 nach einer weiteren Ausführungsform. Bei dieser Ausführungsform ist das distale Ende des Innenschafts 2, das axial über das distale Ende des Außenschafts 3 hinausragt, aus einem Memory-Material ausgebildet, dessen Ruhestellung in Bezug zu der Mittellinie M des hohlen Außenschafts 3 gestreckt oder abgewinkelt ist. Die Zuleitungen der HF-Elektrode sind zweckmäßig unter einem vergleichsweise geringen Abstand zur Innenoberfläche des Innenschafts 2 geführt, sodass bereits relativ kleine Formänderungen des Innenschafts dazu führen, dass die Zuleitungen der HF-Elektrode in Anlage mit der Innenseite des Innenschafts 2 gelangen und die HF-Elektrode beim weiteren Verformen des Memory-Materials geeignet verstellt wird. Durch Anlegen einer elektrischen Spannung, geeignete Temperaturänderung oder Änderung weiterer physikalischer Parameter des Memory-Materials kann somit die Winkelausrichtung der HF-Elektrode relativ zu der Mittellinie M wie gewünscht kontrolliert verstellt werden.

Alternativ kann der Innenschaft 2 selbst als Teil eines Zug- oder Druckgestänges ausgebildet sein, das bei einer axialen Verstellung eine gewünschte Formänderung des distalen Endes des Innenschafts 2 bewirkt.

Gemäß einer weiteren Ausführungsform können die über das distale Ende des Schafts axial hinausragenden Abschnitte der HF-Elektrode selbst unmittelbar aus einem Memorymetall, insbesondere einer elektrisch leitenden Formgedächtnislegierung, ausgebildet sein, sodass durch Anlegen einer elektrischen Spannung, geeignete Temperaturänderung oder Änderung weiterer physikalischer Parameter des Memorymetalls die Winkelausrichtung der HF-Elektrode relativ zu der Mittellinie geeignet verstellt werden kann.

Insgesamt kann nach den vorstehenden Ausführungsformen eine kontrollierte Verstellung der Winkelausrichtung der HF-Elektrode relativ zu der Mittellinie erzielt werden. Im Zusammenwirken mit einer axialen Verstellung des medizinischen Instruments lässt sich so erfindungsgemäß eine praktisch beliebige Positionierung der HF-Elektrode an Positionen auch entfernt zur Mittellinie erzielen, um dort gezielt Gewebe abzutragen. Beschränkungen von herkömmlichen medizinischen Instrumenten, die insbesondere durch das vergleichsweise starre Material von Leitungsabschnitten der HF-Elektrode bedingt waren, können durch die erfindungsgemäß Ausgestaltung überwunden werden.

Wie beispielhaft in der Fig. 5 gezeigt, wird durch die HF-Schlinge in der abgebogenen oder abgewinkelten Arbeitsstellung eine neue Arbeitsachse A gebildet, die unter einem spitzen Winkel W relativ zu der Mittellinie M geneigt ist. Gemäß einer bevorzugten Verwendung bei der transurethralen Resektion ist das medizinische Instrument als Resektoskop ausgebildet, das in einer Einführstellung der HF-Elektrode über den Harnkanal in den Operationsbereich eingeführt wird. Anschließend wird die HF-Elektrode bei geeigneter axialer Stellung in eine Arbeitsstellung mit geeignetem Neigungswinkel W der Arbeitsachse A relativ zur Mittellinie M überführt, wie in der Fig. 5b gezeigt. Dies kann auch eine axiale Verstellung der HF-Elektrode bedingen. Wie dem Fachmann ohne weiteres ersichtlich sein wird, kann in der Arbeitsstellung durch geeignete Längsverstellung des Schafts oder der HF-Elektrode stets eine Stellung gefunden werden, in der das distale Ende der HF-Elektrode abzutragende Gewebeabschnitte im Bereich des Blasenhalses in ausreichendem Maße erreicht bzw. berührt. Durch axiales Verstellen des Schafts oder der HF-Elektrode axial entlang der vorgenannten Einführachse und/oder Änderung des Neigungswinkels der HF-Elektrode relativ zu dem Schaft bzw. der Einführachse können so schrittweise praktisch beliebige Bereiche im Operationsbereich abseits der Einführachse erreicht werden. Wie man der Fig. 6 ohne weiteres entnehmen kann, gilt dies in entsprechender Weise auch für Gewebeabschnitte im Bereich der Prostata, die abseits der vorgenannten Einführachse gelegen sind. Die Reichweite des erfindungsgemäßen Resektoskops ist dabei im Wesentlichen nur durch die Größe der Winkelverstellung der HF-Elektrode und die Distanz beschränkt, um die das distale Ende der HF-Elektrode radial über das Außenprofil des Schafts hinausragen kann.

### Bezugszeichenliste

- 1: Medizinisches Instrument
- 2: Innenschaft
- 3: Außenschaft
- 4: Platziergriff
- 5: Operationsgriff
- 6: Distales Ende

- 10: HF-Elektrode
- 11: Isolationskanal (zur elektrischen Isolation)
- 12: HF-Schlinge
- 15: Verbindungsabschnitt
- 16: seitliche Abtragekante
- 17: symmetrisch gekrümmte Abtragekante
- 18: Gelenk

- 20: Abwinklungshebel

- 100: Harnblase
- 101: Öffnung des Harnleiters
- 102: Bereich des inneren Schließmuskels
- 103: Bereich des äußeren Schließmuskels
- 104: Bereich der Prostata
- 105: Mucosa
- 106: Trigonum vesicae
- 107: Blasenhals

- 110: Schaft
- 111: HF-Elektrode

- M: Mittellinie (des Schafts)
- W: Winkel
- A: Arbeitsflächenmittellinie

## Patentansprüche

1. Medizinisches Instrument (1) zum minimalinvasiven Abtragen von Gewebe mittels einer HF-Elektrode (10), das medizinische Instrument aufweisend die HF-Elektrode (10);
einen hohlen Schaft (2, 3), wobei die HF-Elektrode (10) eine HF-Schlinge (12) und einen Isolationskanal (11) aufweist, wobei der Isolationskanal (11) die HF-Schlinge (12) gegen den hohlen Schaft (2, 3) elektrisch isoliert, wobei die HF-Elektrode (10) an einem distalen Ende (6) des hohlen Schafts (2, 3) angeordnet ist und der Isolationskanal (11) zum Abtragen von Gewebe axial über das distale Ende des Schafts (2, 3) hinausragt, und ein distales Ende der HF-Schlinge (12) zum Abtragen von Gewebe axial über das distale Ende des Isolationskanals (11) hinausragt; die Winkelausrichtung der HF-Elektrode (10) relativ zu dem hohlen Schaft (2, 3) kontrolliert in eine Arbeitsstellung verstellbar ist, in der die HF-Elektrode, in einer Vorderansicht auf den hohlen Schaft (2, 3) betrachtet, zum Abtragen von Gewebe radial über einen Außenumfang des hohlen Schafts hinausragt,
wobei das distale Ende der HF-Schlinge (12) als eine seitliche Abtragekante (16) ausgebildet ist, die über eine symmetrisch gekrümmte Abtragekante (17) in einen Verbindungsabschnitt (15) zur Beaufschlagung der HF-Schlinge (12) mit einer Hochfrequenzspannung übergeht, wobei die Winkelausrichtung des Verbindungsabschnitts (15) relativ zu einer Mittellinie (M) des hohlen Schafts (2, 3) kontrolliert verstellbar ist, um die Winkelausrichtung der HF-Schlinge (12) relativ zu dem hohlen Schaft (2, 3) kontrolliert zu verstellen,
wobei am distalen Ende des hohlen Schafts (2, 3) ein Umlenkelement (20) vorgesehen ist, an dem der Verbindungsabschnitt oder der elektrische Isolationskanal unmittelbar anliegt, wobei das Umlenkelement (20) dergestalt ausgebildet ist, dass durch Längsverschiebung der HF-Elektrode (10) gegen den hohlen Schaft (2, 3) ein zunehmendes Abbiegen oder Abknicken des Verbindungsabschnitts oder des elektrischen Isolationskanals bewirkt wird,
wobei das Umlenkelement (20) als Abwinklungshebel ausgeführt ist, bei dem das distale Ende um eine Achse rotiert werden kann und/oder bei dem eine Winkelstellung kontinuierlich verändert werden kann und/oder das gegen den hohlen Schaft (2, 3) drehbeweglich gelagert ist; oder
das Umlenkelement (20) als bogenförmig gekrümmter Abwinklungsvorsprung auf dem Innenumfang des hohlen Schafts (2, 3) ausgeführt ist; oder
das Umlenkelement (20) als piezoelektrisches Element oder als mechanisch oder thermisch verstellbares Element ausgeführt ist.

2. Medizinisches Instrument (1) nach Anspruch 1, wobei der Verbindungsabschnitt (15) aus einem flexiblen oder elastischen, elektrisch leitenden Material besteht und die Winkelausrichtung der HF-Schlinge (12) relativ zu dem hohlen Schaft (2, 3) durch Biegen oder Abknicken des Verbindungsabschnitts (15) kontrolliert verstellbar ist.

3. Medizinisches Instrument (1) nach Anspruch 2, wobei die HF-Elektrode (10) in die Arbeitsstellung bevorzugt elastisch vorgespannt ist, in der ein distales Ende des Verbindungsabschnitts (15) relativ zu einem proximalen Ende des Verbindungsabschnitts abgebogen oder abgeknickt ist, um mit der Mittellinie (M) des hohlen Schafts (2, 3) einen spitzen Winkel einzuschließen.

4. Medizinisches Instrument (1) nach Anspruch 3, wobei die HF-Elektrode (10) gegen den hohlen Schaft (2, 3) längsverschieblich geführt ist, wobei durch Verstellen der HF-Elektrode (10) hin zum proximalen Ende des hohlen Schafts die HF-Elektrode (10) in eine Einführstellung verstellbar ist, in der das distale Ende des Verbindungsabschnitts (15) mit dem proximalen Ende des Verbindungsabschnitts im Wesentlichen fluchtet und die HF-Schlinge (12) bevorzugt nicht über ein von Innenseiten des hohlen Schafts ausgebildetes Innenprofil hinausragt.

5. Medizinisches Instrument (1) nach Anspruch 4, wobei der Verbindungsabschnitt (15) relativ zu dem elektrischen Isolationskanal (11) oder der elektrische Isolationskanal (11) relativ zu dem hohlen Schaft (2, 3) längsverschieblich geführt ist.

6. Medizinisches Instrument (1) nach Anspruch 4, wobei der Verbindungsabschnitt (15) in einem Überrohr (2) längsverschieblich geführt ist, das relativ zu der Mittellinie (M) des hohlen Schafts (2, 3) bogenförmig gekrümmt ist oder durch Verstellung bogenförmig gekrümmt werden kann, sodass die Position und Winkelausrichtung der HF-Schlinge (12) relativ zu dem hohlen Schaft (2, 3) durch eine Längsverstellung kontrolliert verstellbar ist, um den Verbindungsabschnitt (15) abzubiegen oder abzuknicken.

7. Medizinisches Instrument (1) nach Anspruch 6, wobei das Überrohr (2) unmittelbar als Abschnitt des Isolationskanals (11) ausgebildet ist.

8. Medizinisches Instrument (1) nach Anspruch 6, wobei das Überrohr (2) als hohle Zug- oder Druckstange ausgebildet ist, die mit der HF-Elektrode gekoppelt ist.

9. Medizinisches Instrument (1) nach Anspruch 6, wobei das Überrohr (2) aus einem Memory-Material ausgebildet ist, dessen Ruhestellung in Bezug zu der Mittellinie (M) des hohlen Schafts (2, 3) gestreckt oder abgewinkelt ist, wobei die Orientierung eines distalen Endes des Überrohrs relativ zu der Mittellinie (M) des hohlen Schafts (2, 3) verstellbar ist, insbesondere durch Anlegen eines elektrischen Stroms oder eine Temperaturänderung.

10. Medizinisches Instrument (1) nach einem der Ansprüche 5 bis 9, wobei die HF-Elektrode (10) Gelenke (18) aufweist, die an dem Verbindungsabschnitt (15) angeordnet sind, sodass die Winkelausrichtung der HF-Schlinge (12) relativ zu dem hohlen Schaft (2, 3) durch Verstellen der Gelenke (18) kontrolliert verstellbar ist.

11. Medizinisches Instrument (1) nach Anspruch 10, weiterhin umfassend eine Zug-oder Druckstange, die mit der HF-Schlinge (12) so gekoppelt ist, dass eine Verstellung der Zug- oder Druckstange eine Verstellung der Winkelausrichtung der HF-Schlinge relativ zu dem hohlen Schaft bewirkt.

12. Medizinisches Instrument (1) nach Anspruch 10 oder 11, wobei den Gelenken (18) elastische Rückstellmittel zugeordnet sind, um die HF-Schlinge (12) in eine gestreckte oder abgewinkelte Ruhestellung elastisch vorzuspannen.

13. Medizinisches Instrument (1) nach einem der vorhergehenden Ansprüche, wobei die HF-Elektrode (10) zumindest abschnittsweise aus einem Memorymetall ausgebildet ist, das ausgelegt ist, sodass die Winkelausrichtung der HF-Schlinge (12) relativ zu dem hohlen Schaft (2, 3) kontrolliert verstellbar ist, insbesondere durch Anlegen eines elektrischen Stroms oder eine Temperaturänderung.

14. Medizinisches Instrument (1) nach einem der vorherigen Ansprüche, wobei der hohle Schaft einen hohlen Außenschaft (3) und einen darin aufgenommenen Innenschaft (2) aufweist, wobei der Innenschaft (2) in dem hohlen Außenschaft (3) längsverschieblich geführt ist und wobei die HF-Elektrode (10) vollständig in den hohlen Außenschaft (3) zurückgefahren werden kann.

15. Medizinisches Instrument (1) nach einem der vorherigen Ansprüche, wobei ein Neigungswinkel der HF-Elektrode (10) relativ zu der Mittellinie (M) des hohlen Schafts (2, 3) um bis zu 90° verstellbar ist, bevorzugter in einem Bereich von 50° bis 75°.

## Claims

1. Medical instrument (1) for minimally invasive removal of tissue by means of a HF electrode (10), the medical instrument comprising the HF electrode (10); a hollow shaft (2, 3), wherein
the HF electrode (10) has a HF loop (12) and an insulation channel (11), wherein the insulation channel (11) electrically insulates the HF loop (12) from the hollow shaft (2, 3), wherein the HF electrode (10) is arranged at a distal end (6) of the hollow shaft (2, 3) and the insulation channel (11) protrudes axially beyond the distal end of the shaft (2, 3) for removing tissue, and a distal end of the HF loop (12) protrudes axially beyond the distal end of the insulation channel (11) for removing tissue;
the angular orientation of the HF electrode (10) relative to the hollow shaft (2, 3) can be adjusted in a controlled manner into a working position in which the HF electrode, in a front view of the hollow shaft (2, 3), projects radially beyond an outer circumference of the hollow shaft for removing tissue,
wherein the distal end of the HF loop (12) is designed as a lateral removal edge (16) which transitions via a symmetrically curved removal edge (17) into a connecting portion (15) for applying a high-frequency voltage to the HF loop (12), wherein the angular orientation of the connecting portion (15) relative to a center line (M) of the hollow shaft (2,3) can be adjusted in a controlled manner in order to adjust the angular orientation of the HF loop (12) relative to the hollow shaft (2,3) in a controlled manner,
wherein a deflecting element (20) against which the connecting portion or the electrical insulation channel bears directly is provided at the distal end of the hollow shaft (2, 3), wherein the deflecting element (20) is designed in such a way that longitudinal displacement of the HF electrode (10) with respect to the hollow shaft (2, 3) causes increasing bending or kinking of the connecting portion or of the electrical insulation channel, wherein the deflecting element (20) is designed as a bending lever, whereby the distal end can be rotated about an axis and/or whereby an angular position can be continuously changed and/or which is mounted so as to be rotatable with respect to the hollow shaft (2, 3); or the deflecting element (20) is designed as an arcuately curved bending projection on the inner circumference of the hollow shaft (2, 3); or
the deflecting element (20) is designed as a piezoelectric element or as a mechanically or thermally adjustable element.

2. Medical instrument (1) according to claim 1, wherein the connecting portion (15) consists of a flexible or resilient electrically conductive material and the angular orientation of the HF loop (12) relative to the hollow shaft (2,3) can be adjusted in a controlled manner by bending or kinking the connecting portion (15).

3. Medical instrument (1) according to claim 2, wherein the HF electrode (10) is preferably resiliently preloaded into the working position, in which a distal end of the connecting portion (15) is bent or kinked relative to a proximal end of the connecting portion in order to form an acute angle with the center line (M) of the hollow shaft (2,3).

4. Medical instrument (1) according to claim 3, wherein the HF electrode (10) is guided so as to be longitudinally displaceable with respect to the hollow shaft (2, 3), wherein, by adjusting the HF electrode (10) towards the proximal end of the hollow shaft, the HF electrode (10) can be adjusted into an insertion position in which the distal end of the connecting portion (15) is substantially aligned with the proximal end of the connecting portion and the HF loop (12) preferably does not protrude beyond an inner profile formed by inner sides of the hollow shaft,

5. Medical instrument (1) according to claim 4, wherein the connecting portion (15) is guided so as to be longitudinally displaceable relative to the electrical insulation channel (11) or the electrical insulation channel (11) is guided so as to be longitudinally displaceable relative to the hollow shaft (2, 3).

6. Medical instrument (1) according to claim 4, wherein the connecting portion (15) is guided so as to be longitudinally displaceable in a cover tube (2) which is arcuately curved relative to the center line (M) of the hollow shaft (2, 3) or can be arcuately curved by adjustment, so that the position and angular orientation of the HF loop (12) relative to the hollow shaft (2, 3) can be adjusted in a controlled manner by a longitudinal adjustment in order to bend or kink the connecting portion (15).

7. Medical instrument (1) according to claim 6, wherein the cover tube (2) is formed directly as a portion of the insulation channel (11).

8. Medical instrument (1) according to claim 6, wherein the cover tube (2) is designed as a hollow pull or push rod which is coupled to the HF electrode,

9. Medical instrument (1) according to claim 6, wherein the cover tube (2) is formed from a memory material of which the idle position is stretched or angled in relation to the center line (M) of the hollow shaft (2,3), wherein the orientation of a distal end of the cover tube relative to the center line (M) of the hollow shaft (2, 3) is adjustable, in particular by applying an electric current or a temperature change.

10. Medical instrument (1) according to any of claims 5 to 9, wherein the HF electrode (10) has joints (18) arranged on the connecting portion (15) so that the angular orientation of the HF loop (12) relative to the hollow shaft (2, 3) can be adjusted in a controlled manner by adjusting the joints (18).

11. Medical instrument (1) according to claim 10,
further comprising a pull or push rod coupled to the HF loop (12) such that an adjustment of the pull or push rod causes an adjustment of the angular orientation of the HF loop relative to the hollow shaft,

12. Medical instrument (1) according to claim 10 or 11,
wherein the joints (18) are associated with resilient return means in order to resiliently preload the HF loop (12) into a stretched or angled idle position.

13. Medical instrument (1) according to any of the preceding claims, wherein the HF electrode (10) is formed at least partly from a memory metal which is designed so that the angular orientation of the HF loop (12) relative to the hollow shaft (2, 3) can be adjusted in a controlled manner, in particular by applying an electric current or a temperature change.

14. Medical instrument (1) according to any of the preceding claims, wherein the hollow shaft has a hollow outer shaft (3) and an inner shaft (2) received therein, wherein the inner shaft (2) is guided so as to be longitudinally displaceable in the hollow outer shaft (3) and wherein the HF electrode (10) can be completely retracted into the hollow outer shaft (3).

15. Medical instrument (1) according to any of the preceding claims, wherein an angle of inclination of the HF electrode (10) relative to the center line (M) of the hollow shaft (2, 3) is adjustable by up to 90°, more preferably in a range of from 50° to 75°.

## Revendications

1. Instrument médical (1) pour l'ablation peu invasive de tissu au moyen d'une électrode HF (10), l'instrument médical présentant l'électrode HF (10) ; une tige creuse (2, 3), dans lequel
l'électrode HF (10) présente une boucle HF (12) et un canal d'isolation (11), dans lequel le canal d'isolation (11) isole électriquement la boucle HF (12) de la tige creuse (2, 3), dans lequel l'électrode HF (10) est disposée à une extrémité distale (6) de la tige creuse (2, 3) et le canal d'isolation (11) fait saillie axialement au-delà de l'extrémité distale de la tige (2, 3) pour l'ablation de tissu, et une extrémité distale de la boucle HF (12) fait saillie axialement au-delà de l'extrémité distale du canal d'isolation (11) pour l'ablation de tissu ;
l'orientation angulaire de l'électrode HF (10) par rapport à la tige creuse (2, 3) est réglable de manière contrôlée dans une position de travail dans laquelle l'électrode HF, vue de face sur la tige creuse (2, 3), fait saillie radialement au-delà d'une périphérie extérieure de la tige creuse pour l'ablation de tissu,
dans lequel l'extrémité distale de la boucle HF (12) est conçue comme une arête d'ablation (16) latérale
qui se transforme, par l'intermédiaire d'une arête d'ablation (17) courbée de manière symétrique, en une section de liaison (15) pour la sollicitation de la boucle HF (12) par une tension haute fréquence, dans lequel l'orientation angulaire de la section de liaison (15) est réglable de manière contrôlée par rapport à une ligne centrale (M) de la tige creuse (2, 3) afin de régler de manière contrôlée l'orientation angulaire de la boucle HF (12) par rapport à la tige creuse (2, 3),
dans lequel un élément de déviation (20) est prévu au niveau de l'extrémité distale de la tige creuse (2, 3), sur lequel la section de liaison ou le canal d'isolation électrique s'appuie directement, dans lequel l'élément de déviation (20) est réalisé de telle sorte que, au moyen du déplacement longitudinal de l'électrode HF (10) contre la tige creuse (2, 3), une pliure ou un fléchissement croissant de la section de liaison ou du canal d'isolation électrique est provoqué, dans lequel l'élément de déviation (20) est conçu sous forme de levier de pliage dont l'extrémité distale peut tourner autour d'un axe et/ou dont une position angulaire peut être modifiée en continu et/ou lequel levier de pliage est monté de manière mobile en rotation contre la tige creuse (2, 3) ; ou l'élément de déviation (20) est conçu sous forme de saillie de pliage courbée en forme d'arc sur la périphérie intérieure de la tige creuse (2, 3) ; ou
l'élément de déviation (20) est conçu sous forme d'élément piézoélectrique ou d'élément réglable mécaniquement ou thermiquement.

2. Instrument médical (1) selon la revendication 1, dans lequel la section de liaison (15) est constituée d'un matériau flexible ou élastique et électriquement conducteur, et l'orientation angulaire de la boucle HF (12) par rapport à la tige creuse (2, 3) est réglable de manière contrôlée en pliant ou en fléchissant la section de liaison (15).

3. Instrument médical (1) selon la revendication 2, dans lequel l'électrode HF (10) est précontrainte, de préférence de manière élastique, dans la position de travail dans laquelle une extrémité distale de la section de liaison (15) est pliée ou fléchie par rapport à une extrémité proximale de la section de liaison afin de former un angle aigu avec la ligne centrale (M) de la tige creuse (2, 3).

4. Instrument médical (1) selon la revendication 3, dans lequel l'électrode HF (10) est guidée de manière à pouvoir se déplacer longitudinalement contre la tige creuse (2, 3), dans lequel, en réglant l'électrode HF (10) vers l'extrémité proximale de la tige creuse, l'électrode HF (10) est réglable dans une position d'introduction, dans laquelle l'extrémité distale de la section de liaison (15) est sensiblement alignée avec l'extrémité proximale de la section de liaison et la boucle HF (12) ne fait de préférence pas saillie au-delà d'un profil intérieur réalisé par les côtés intérieurs de la tige creuse.

5. Instrument médical (1) selon la revendication 4, dans lequel la section de liaison (15) est guidée de manière à pouvoir se déplacer longitudinalement par rapport au canal d'isolation (11) électrique ou le canal d'isolation (11) électrique est guidé de manière à pouvoir se déplacer longitudinalement par rapport à la tige creuse (2, 3).

6. Instrument médical (1) selon la revendication 4, dans lequel la section de liaison (15) est guidée de manière à pouvoir se déplacer longitudinalement dans un surtube (2) qui est courbé en forme d'arc par rapport à la ligne centrale (M) de la tige creuse (2, 3) ou qui peut être courbé en forme d'arc par réglage, de sorte que la position et l'orientation angulaire de la boucle HF (12) par rapport à la tige creuse (2, 3) sont réglables de manière contrôlée par un réglage longitudinal afin de plier ou de fléchir la section de liaison (15).

7. Instrument médical (1) selon la revendication 6, dans lequel le surtube (2) est réalisé directement sous forme de section du canal d'isolation (11).

8. Instrument médical (1) selon la revendication 6, dans lequel le surtube (2) est réalisé sous forme de tige de traction ou de compression creuse qui est accouplée à l'électrode HF.

9. Instrument médical (1) selon la revendication 6, dans lequel le surtube (2) est réalisé à partir d'un matériau à mémoire de forme dont la position de repos est étirée ou coudée par rapport à la ligne centrale (M) de la tige creuse (2, 3), dans lequel l'orientation d'une extrémité distale du surtube est réglable par rapport à la ligne centrale (M) de la tige creuse (2, 3), en particulier par application d'un courant électrique ou d'une variation de température.

10. Instrument médical (1) selon l'une des revendications 5 à 9, dans lequel l'électrode HF (10) présente des articulations (18) qui sont disposées sur la section de liaison (15), de sorte que l'orientation angulaire de la boucle HF (12) par rapport à la tige creuse (2, 3) est réglable de manière contrôlée par réglage des articulations (18).

11. Instrument médical (1) selon la revendication 10,
comprenant en outre une tige de traction ou de compression qui est accouplée à la boucle HF (12) de sorte qu'un réglage de la tige de traction ou de compression provoque un réglage de l'orientation angulaire de la boucle HF par rapport à la tige creuse.

12. Instrument médical (1) selon la revendication 10 ou 11,
dans lequel des moyens de rappel élastiques sont associés aux articulations (18) pour précontraindre élastiquement la boucle HF (12) dans une position de repos étirée ou coudée.

13. Instrument médical (1) selon l'une des revendications précédentes, dans lequel l'électrode HF (10) est réalisée au moins dans certaines sections à partir d'un métal à mémoire de forme qui est conçu de sorte que l'orientation angulaire de la boucle HF (12) par rapport à la tige creuse (2, 3) est réglable de manière contrôlée, en particulier par l'application d'un courant électrique ou d'une variation de température.

14. Instrument médical (1) selon l'une des revendications précédentes, dans lequel la tige creuse présente une tige extérieure creuse (3) et une tige intérieure (2) logée dans celle-ci, dans lequel la tige intérieure (2) est guidée de manière à pouvoir se déplacer longitudinalement dans la tige extérieure creuse (3) et dans lequel l'électrode HF (10) peut être entièrement rétractée dans la tige extérieure creuse (3).

15. Instrument médical (1) selon l'une des revendications précédentes, dans lequel un angle d'inclinaison de l'électrode HF (10) par rapport à la ligne centrale (M) de la tige creuse (2, 3) est réglable jusqu'à 90°, de préférence dans une plage allant de 50° à 75°.
